# EUROPÄISCHE PATENTSCHRIFT

(11) **EP 1 387 653 B1**
(45) Veröffentlichungstag und Bekanntmachung des Hinweises auf die Patenterteilung: **02.11.2005**
(21) Anmeldenummer: 02738074.0
(22) Anmeldetag: 15.05.2002
(51) Int. Cl.: A61F 9/013

(54) **MARKIERUNGSINSTRUMENT**
MARKING INSTRUMENT
INSTRUMENT DE MARQUAGE

(30) Priorität: 18.05.2001 DE 10124708
(43) Veröffentlichungstag der Anmeldung: 11.02.2004
(73) Patentinhaber: Gerten, Georg, 53121 Bonn (DE)
(72) Erfinder: Gerten, Georg, 53121 Bonn (DE)
(74) Vertreter: Buschhoff, Josef
(86) Internationale Anmeldenummer: PCT/EP2002/005349
(87) Internationale Veröffentlichungsnummer: WO 2002/094144

(56) Entgegenhaltungen:
- EP-A- 0 535 378
- US-A- 5 102 391
- US-A- 5 956 260
- US-B1- 6 217 596

## Beschreibung

Die Erfindung betrifft ein Markierungsinstrument zum Erzeugen von, insbesondere horizontalen und/oder vertikalen, Markierungen für augenchirurgische Anwendungen gemäß Anspruch 1, mit einem Markierungselemente aufweisenden, gegen das zu markierende Objekt anstellbaren Instrumentenkopf und einem daran angeordneten Instrumentengriff.

Vor augenchirurgischen Eingriffen ist es häufig erforderlich, am späteren Operationsfeld Markierungen anzubringen, während der Patient steht oder sitzt, so daß während der im allgemeinen im Liegen des Patienten erfolgenden Operation der Operateur wichtige Bezugspunkte zur Verfügung hat, um die für die Operation erforderlichen chirurgischen Eingriffe an der richtigen Stelle und in richtiger Orientierung vornehmen zu können. Besonders wichtig sind solche Markierungen bei chirurgischen Eingriffen am Auge, wo es beim Einsetzen von intraokularen Linsen, in der Hornhautchirurgie oder der refraktiven Chirurgie häufig darauf ankommt, dem Operateur die korrekte horizontale oder vertikale Achse des Auges anzuzeigen.

Bislang werden derartige Markierungen mit einem Markierungsinstrument frei Hand auf dem zu markierenden Objekt angebracht, indem die Markierungselemente des verwendeten Markierungsinstruments zunächst ähnlich wie ein Stempel mit Markierungstinte versehen und dann gegen die zu markierende Oberfläche, beispielsweise die Hornhaut eines zu operierenden Auges gedrückt werden. Hierbei steht oder sitzt der Patient weitgehend aufrecht, wobei er beim Markieren der Hornhaut des Auges den Kopf gerade halten und ein auf Augenhöhe in einem bestimmten Abstand angeordnetes Objekt fixieren soll. Der Operateur richtet dann das Markierungsinstrument nach Augenmaß so aus, daß die Markierungselemente die gewünschte Lage einnehmen, also insbesondere horizontal und/oder vertikal stehen, bevor er den Instrumentenkopf gegen die zu markierende Fläche drückt, auf der dann die zuvor mit Markierungstinte versehenen Markierungselemente die gewünschte Markierung hinterlassen.

Die Ausrichtung des Markierungsinstruments allein von Hand nach Augenmaß führt regelmäßig dazu, daß die Genauigkeit beim Markieren zu wünschen übrig läßt und somit eine beispielsweise vertikale oder horizontale Achse am Auge vor hornhautchirurgischen Eingriffen nie 100%-ig definiert werden kann, sondern immer von dem Augenmaß der Person abhängt, die die Markierung anbringt.

Ein Instrument gemäß dem Oberbegriff des Anspruchs 1 ist aus der US 6 217 596 bekannt.

Aufgabe der Erfindung ist es, diesen Nachteil zu vermeiden und ein Markierungsinstrument zu schaffen, das es ermöglicht, Markierungen an einem Operationgsgebiet in exakt der gewünschten, vorzugsweise horizontalen und/oder vertikalen Ausrichtung anzubringen.

Diese Aufgabe wird mit der Erfindung gemäß Anspruch 1 gelöst.

Gemäß der Erfindung weist, die Positioniereinrichtung einen schwerkraftbelasteten, den Instrumentenkopf relativ zum Instrumentengriff einstellenden Einstellmechanismus auf, der beispielsweise ein am Instrumentenkopf wirksames Lotgewicht sowie ein Schwenklager haben kann, um das sich der Instrumentenkopf relativ zum Instrumentengriff verdrehen kann. Bei dieser Ausgestaltung der Erfindung stellt sich der Instrumentenkopf automatisch in die gewünschte Winkelstellung relativ zur Erdnormalen ein, und zwar unabhängig davon, in welcher Winkelstellung der Instrumentengriff gehalten wird. Die die Markierung am Operationsfeld anbringende Person muß lediglich den Instrumentengriff solange ruhig halten, bis das Lotgewicht von der Erdanziehungskraft in die tiefste, mögliche Position gezogen wurde und dadurch den Instrumentenkopf in die korrekte Lage ausgerichtet hat. Vorzugsweise ist dem Schwenklager hierbei ein Dämpfungselement zugeordnet, das verhindert, daß der Instrumentenkopf mit seinem Lotgewicht erst nach mehrmaligem Hin- und Herpendeln in die statisch stabile Lage gelangt, in der sich das Lotgewicht in seiner tiefstmöglichen Lage befindet.

Weitere Merkmale und Vorteile der Erfindung ergeben sich aus der nachfolgenden Beschreibung und der Zeichnung, worin bevorzugte Ausführungsformen der Erfindung anhand von Beispielen näher erläutert werden. Es zeigt:
- **Fig. 1**: eine Ausführungsform eines nicht erfindungsgemäßen Markierungsinstruments zum Anbringen von Markierungen auf der Hornhaut eines Auges in einer Ansicht;
- **Fig. 2**: den Gegenstand der Fig. 1 im Schnitt längs der Linie II-II;
- **Fig. 3**: eine zweite Ausführungsform des erfindungsgemäßen Markierungsinstruments in einer Ansicht; und
- **Fig. 4**: den Gegenstand der Fig. 3 im Schnitt längs der Linie IV-IV.

Die in den Zeichnungen dargestellten Markierungsinstrumente 10 dienen dazu, auf der Hornhaut eines Auges eines Patienten, der sich einem augenchirurgischen Eingriff unterziehen will, Markierungen anzuzeichnen, die dem Operateur die horizontale und/oder vertikale Achsposition des Auges anzeigen. Die Markierungen werden mit Hilfe des Markierungsinstruments 10 ähnlich wie ein Stempelabdruck auf die Hornhaut des Auges übertragen, während der Patient steht oder aufrecht sitzt, wobei er den Kopf gerade halten und mit dem zu markierenden Auge einen vorher definierten Fluchtpunkt anpeilen soll, während die die Markierung vornehmende Person das Markierungsinstrument in die korrekte Lage ausrichtet und gegen die Hornhaut des Patienten anstellt.

Wie sich aus den Zeichnungen ergibt, besteht das Markierungsinstrument im wesentlichen aus einem Instrumentengriff 11 und einem daran angeordneten Instrumentenkopf 12, der an seiner dem zu markierenden Auge zugewandten Vorderseite 13 insgesamt vier im gleichmäßigen Winkelabstand voneinander angeordnete Markierungselemente 14 aufweist, die an ihrer freien Vorderseite an die Kontur des zu markierenden Auges angepaßt sind. Die Markierungselemente bilden mit ihren Vorderkanten 15 Stempelflächen, die mit einer geeigneten Stempeltinte od. dgl. eingefärbt werden können, die auf die Hornhaut des Patienten übertragen wird, wenn die Markierungselemente in Kontakt mit dem Patientenauge kommen.

Bei den dargestellten Ausführungsbeispielen sollen Markierungen für die horizontale und vertikale Achsposition auf der Hornhaut des zu operierenden Auges 16 angebracht werden; demgemäß ist der Instrumentenkopf mit zwei horizontalen Markierungselementen 14a und zwei vertikalen Markierungselementen 14b versehen, die während des Markierungsvorgangs möglichst genau senkrecht bzw. parallel zur Erdnormalen ausgerichtet sein sollen, um die horizontale bzw. vertikale Achse auf der Hornhaut des Patienten genau zu definieren. Hierzu ist das erfindungsgemäße Markierungsinstrument 10 mit einer die gewünschte Winkelstellung der Markierungselemente relativ zur Erdnormalen 17 anzeigenden bzw. einstellenden Positioniereinrichtung versehen, die es der die Markierung anbringenden Person ermöglicht, dies mit großer Genauigkeit durchzuführen.

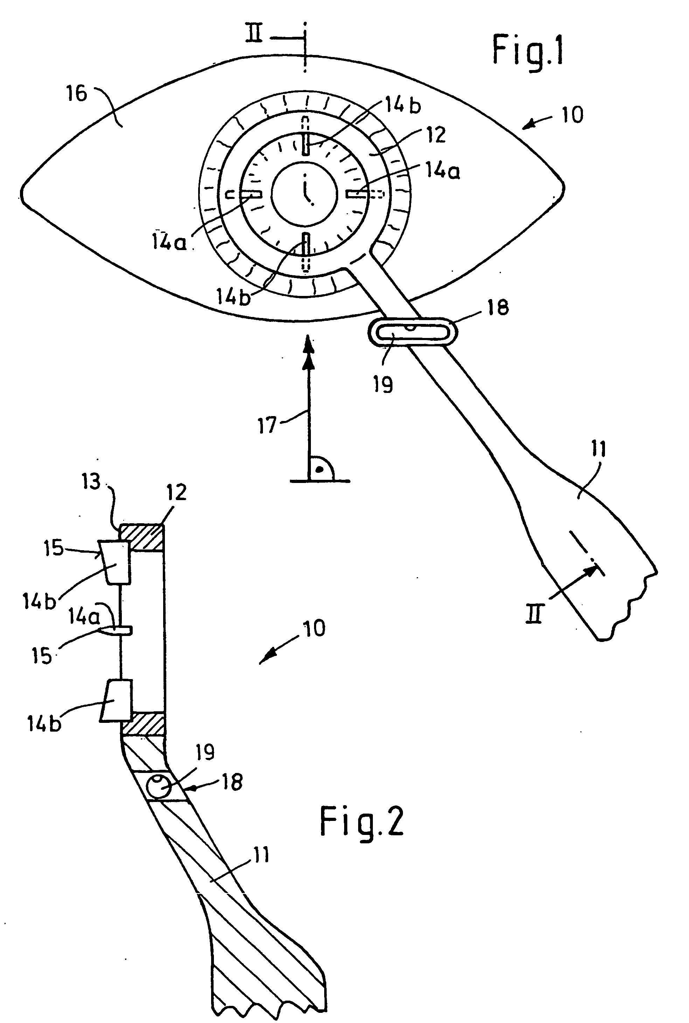

Bei der in den Fig. 1 und 2 dargestellten, nicht zur Erfindung gehörenden, Ausführungsform besteht die Positioniereinrichtung im wesentlichen aus einer Libelleneinrichtung 18 mit einem bis auf eine Gasblase mit Flüssigkeit gefüllten Schauglas 19, das am Instrumentengriff 11 in der Nähe des Instrumentenkopfes 12 angeordnet ist und dem Benutzer ähnlich wie eine Wasserwaage anzeigt, in welcher Stellung des Instrumentengriffes 11 der Instrumentenkopf mit den daran fest angebrachten Markierungselementen die korrekte Lage einnimmt, um eine genau horizontale bzw. vertikale Markierung auf der zu markierenden Fläche, also der Hornhaut des Patienten zu erzeugen. Hierzu prüft die die Markierung anbringende Person die Anzeige im Schauglas 19 und hält den Instrumentengriff 11 des Markierungsinstruments in einem solchen Winkel, in dem die Gasblase im Schauglas 19 genau zwischen zwei daran angebrachten Justierstrichen steht. Erst dann wird das Markierungsinstrument gegen die zu markierende Fläche angestellt, ohne dabei die Winkelstellung des Instrumentengriffes relativ zur Erdnormalen nochmals zu verändern. Auf diese Weise ist es mit hoher Genauigkeit möglich, die Markierungen in ihrem gewünschten Winkel relativ zur Erdnormalen anzubringen.

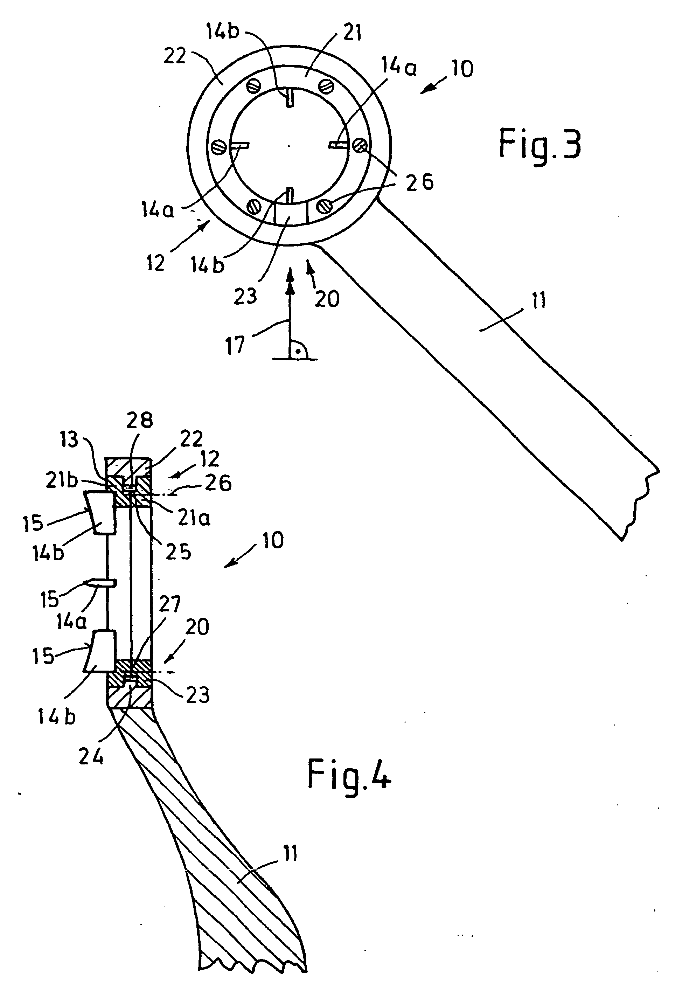

Bei der in den Fig. 3 und 4 dargestellten Ausführungsform weist die Positioniereinrichtung einen den Instrumentenkopf relativ zum Instrumentengriff einstellenden Einstellmechanismus 20 auf, der automatisch für eine korrekte Ausrichtung der Markierungselemente vor dem Anbringen der Markierungen auf der Augenhornhaut sorgt. Hierzu ist der Instrumentenkopf als hochgenau gearbeiteter Drehring 21 ausgestaltet, der in einer am vorderen Ende des Instrumentengriffes angeordneten Aufnahmenabe 22 frei drehbar gelagert ist. Der ansonsten aus einem eher leichten Werkstoff gefertigte Drehring ist in einem schmalen, koaxial zu den beiden vertikalen Markierungselementen 14b verlaufenden Ringsegment mit einem Lotgewicht 23 versehen, das von der in Richtung der Erdnormalen wirkenden Erdanziehungskraft beim Gebrauch des Markierungsinstrumentes immer nach unten gezogen wird. Das Lotgewicht sorgt also dafür, daß die vertikalen Markierungsinstrumente 14b parallel und die horizontalen Markierungselemente 14a rechtwinklig zur Erdnormalen 17 ausgerichtet werden, indem sich der Drehring 21 in der Aufnahmenabe 22 infolge der das Lotgewicht nach unten ziehenden Erdanziehungskraft von selbst in diese gewünschte Position einstellt. Dabei ist es unerheblich, unter welchem Winkel der Instrumentengriff 11 von der Person gehalten wird, die die Markierung auf der zu markierenden Fläche anbringen soll.

Man erkennt aus Fig. 4, daß der Drehring 21 gegen Herausfallen aus der Aufnahmenabe 22 gesichert ist. Hierzu weist die Aufnahmenabe einen umlaufenden, ringförmigen Steg 24 auf, der in eine umlaufende Ringnut 25 am Drehring einfaßt. Zur Montage des Drehringes ist dieser zweiteilig gefertigt, wobei die beiden Teile 21a, 21b von beiden Seiten der Aufnahmenabe 22 in diese eingesetzt und mittels mehrerer Verbindungsschrauben 26 lösbar miteinander verbunden werden. Als Material für den Drehring und die Aufnahmenabe kommen insbesondere Werkstoffe mit sehr geringem Wärmeausdehnungskoeffizient in Frage, beispielsweise keramische Werkstoffe, so daß die freie Drehbarkeit des Drehrings in der Nabe durch Schwankungen der Umgebungstemperatur nicht beeinflußt wird. Um ein zu starkes Pendeln des Drehrings in der Aufnahmenabe zu vermeiden, kann der sich zwischen dem Steg und der Ringnut 25 ausbildende Spalt 27 mit einer Dämpfungsflüssigkeit 28 gefüllt sein, die dann dafür sorgt, daß das Lotgewicht 23 bei seinem Weg in die dargestellte, tiefste Position nicht oder jedenfalls nicht nennenswert an der gegenüberliegenden Seite wieder ausschwingt.

Die Erfindung ist nicht auf die dargestellten und beschriebenen Ausführungsbeispiele beschränkt, sondern es sind verschiedene Änderungen und Ergänzungen denkbar. So muß es sich bei einer zum Einsatz kommenden Libellen- oder Loteinrichtung nicht um das beschriebene, rein opto-mechanische Instrument handeln, sondern es kann ein elektronischer Lotgeber eingesetzt werden, der die korrekte Ausrichtung des Instrumentes anhand einer elektrischen oder elektronischen Anzeige wie beispielsweise einer Leuchtdiode od.dgl. anzeigt. Selbstverständlich ist die Libellen- bzw. Loteinrichtung nicht am Instrumentengriff angeordnet, sondern am Instrumentenkopf des Markierungsinstrumentes, also dem Teil, auf dessen korrekte.Ausrichtung es beim Anbringen der Markierungen tatsächlich ankommt.

## Patentansprüche

1. Markierungsinstrument zum Erzeugen von Markierungen für augenchirurgische Anwendungen, insbesondere von horizontalen und/oder vertikalen Markierungen, mit einem Markierungselemente (14) aufweisenden, gegen das zu markierende Objekt (16) anstellbaren Instrumentenkopf (12) und einem daran angeordneten Instrumentengriff (11), und mit einer die gewünschte Winkelstellung der Markierungselemente (14) relativ zur Erdnormalen (17) einstellenden Positioniereinrichtung (18; 20), **dadurch gekennzeichnet , daß** die Positioniereinrichtung einen schwerkraftbelasteten, den Instrumentenkopf (12) relativ zum Instrumentengriff (11) einstellenden Einstellmechanismus (20) mit einem am Instrumentenkopf (12) wirksamen Lotgewicht (23) sowie einem Schwenklager (21, 22) aufweist um das sich der Instrumentenkopf (12) relativ zum Instrumentengriff (11) verdrehen kann..

2. Markierungsinstrument nach Anspruch 1, **gekennzeichnet durch** ein dem Schwenklager (21, 22) zugeordnetes Dämpfungselement (28).

3. Markierungsinstrument nach Anspruch 1 oder 2, **dadurch gekennzeichnet, daß** die Positioniereinrichtung eine Libellen- oder Loteinrichtung (18) aufweist.

4. Markierungsinstrument nach Anspruch 3, **dadurch gekennzeichnet, daß** die Libellen- oder Loteinrichtung (18) am Instrumentengriff (11) angeordnet ist.

5. Markierungsinstrument nach Anspruch 3 oder 4, **dadurch gekennzeichnet, daß** die Libellen- oder Loteinrichtung (18) eine Anzeige aufweist, die in der Nähe des Instrumentenkopfes (12) angeordnet ist.

6. Markierungsinstrument nach einem der Ansprüche 3 bis 5, **dadurch gekennzeichnet, daß** die Libellen- oder Loteinrichtung (18) im wesentlichen aus einem bis auf eine Gasblase mit Flüssigkeit gefüllten Schauglas (19) besteht.

7. Markierungsinstrument nach einem der Ansprüche 3 bis 5, **dadurch gekennzeichnet, daß** die Libellen- oder Loteinrichtung (18) im wesentlichen aus einem elektronischen Lotgeber und einer vorzugsweise im Bereich des Instrumentenkopfes (12) angeordneten elektrischen bzw. elektronischen Anzeige besteht.

## Claims

1. Marking instrument for producing marks for optical surgery uses, in particular horizontal and/or vertical marks, having an instrument head (12) comprising marking elements (14), adjustable against the object (16) to be marked, and an instrument handle (11) arranged thereon, and with a positioning device (18; 20) adjusting the desired angular position of the marking elements (14) relative to the normal (17) of the earth, **characterised in that** the positioning device comprises a gravity-loaded adjustment mechanism (20) adjusting the instrument head (12) relative to the instrument handle (11) with a verticality weight (23) effective at the instrument head (12), as well as a pivot bearing (21, 22) around which the instrument head (12) can rotate relative to the instrument handle (11).

2. Marking instrument according to claim 1, **characterised by** a dampening element (28) assigned to the pivot bearing (21, 22).

3. Marking instrument according to one of claims 1 to 2, **characterised in that** the positioning device comprises a level or verticality indicator (18).

4. Marking instrument according to claim 3, **characterised in that** the level or verticality indicator (18) is arranged on the instrument handle (11)

5. Marking instrument according to claim 3 or 4, **characterised in that** the level or verticality indicator (18) comprises a display which is arranged in the vicinity of the instrument head (12).

6. Marking instrument according to one of claims 3 to 5, **characterised in that** the level or verticality indicator (18) essentially consists of a sight glass (19) filled with a liquid apart from a gas bubble.

7. Marking instrument according to one of claims 3 to 5, **characterised in that** the level or verticality indicator (18) essentially consists of an electronic verticality encoder and an electric or electronic display arranged preferably in the region of the instrument head (12).

## Revendications

1. Instrument de marquage pour la production de marquages, pour des applications en chirurgie oculaire, en particulier de marquages horizontaux et/ou verticaux, comprenant d'une part une tête (12) de l'instrument, la tête présentant des éléments de marquage (14) et pouvant être mise en place contre l'objet (16) à marquer, et d'autre part un manche (11) de l'instrument, le manche étant agencé sur la tête, ledit instrument comprenant en outre un dispositif de positionnement (18; 20) réglant la position angulaire souhaitée des éléments de marquage (14) par rapport à la normale terrestre (17), **caractérisé en ce que** le dispositif de positionnement présente un mécanisme de réglage (20) chargé par la pesanteur et réglant la position de la tête (12) par rapport au manche (11), le mécanisme comprenant un poids de verticalité (23) agissant sur la tête (12) ainsi qu'un palier de pivotement (21, 22), autour duquel la tête (12) peut tourner par rapport au manche (11).

2. Instrument de marquage selon la revendication 1, **caractérisé en ce qu'**il comprend en outre un élément d'amortissement (28) associé au palier de pivotement (21, 22).

3. Instrument de marquage selon la revendication 1 ou 2, **caractérisé en ce que** le dispositif de positionnement présente un dispositif à effet de niveau ou de fil à plomb (18).

4. Instrument de marquage selon la revendication 3, **caractérisé en ce que** le dispositif à effet de niveau ou de fil à plomb (18) est agencé sur le manche (11).

5. Instrument de marquage selon la revendication 3 ou 4, **caractérisé en ce que** le dispositif à effet de niveau ou de fil à plomb (18) présente un affichage proche de la tête (12).

6. Instrument de marquage selon l'une des revendications 3 à 5, **caractérisé en ce que** le dispositif à effet de niveau ou de fil à plomb (18) consiste pour l'essentiel en un verre de visualisation (19) rempli d'un fluide à l'exception d'une bulle de gaz.

7. Instrument de marquage selon l'une des revendications 3 à 5, **caractérisé en ce que** le dispositif à effet de niveau ou de fil à plomb (18) consiste pour l'essentiel en un capteur électronique de verticalité et en une visualisation électrique ou électronique agencée de préférence dans la région de la tête (12).
